# EUROPEAN PATENT APPLICATION

(11) **EP 4 005 538 A1**
(43) Date of publication of application: **01.06.2022**
(21) Application number: 21209811.5
(22) Date of filing: 23.11.2021
(51) Int. Cl.: A61F 5/01

(54) **3D PRINTED ANKLE AND FOOT ORTHOSIS AND A METHOD OF PRODUCTION OF THE SAME**

(30) Priority: 27.11.2020 CZ 20200636
(71) Applicant: INVENT MEDICAL GROUP, S.R.O., 70800 Ostrava, Pustkovec (CZ)
(72) Inventor: ROSICKY, Jiri, 73911 Frydlant nad Ostravici (CZ); BOUMA, Tomas, 73532 Rychvald (CZ)
(74) Representative: Dadej, Leopold

(57) **Abstract**

An object of the invention is a 3D printed ankle and foot orthosis comprising a 3D printed shell (1), wherein the shell (1) comprises a bottom part (2) and a top part (3), and an anterior piece (9) located between a medial part (4) and a lateral part (5), wherein the anterior piece (9) is adapted to encircle the anterior region (10) of the foot and ankle between the medial part (4) and the lateral part (5), wherein the anterior piece is formed of a single material, wherein the medial side (12) of the anterior piece and the lateral side (13) of the anterior piece each comprise at least one transverse cutout (14) from the margin of the medial side (12) of the anterior piece and the lateral side (13) of the anterior piece towards a central part (11) of the anterior piece, wherein the transverse cutouts (14) are adapted to increase the flexibility of the central part (11) of the anterior piece at the point between the transverse cutouts (14). Furthermore, a method of production of such an orthosis is also an object of the invention.

## Description

### Technical Field

The invention relates to a 3D printed ankle and foot orthosis and a method of production of the same.

### Background of the Invention

For safe and comfortable movement with an ankle and foot orthosis, it is essential that the orthosis is precisely adapted to the anatomical shape of the wearer's ankle and foot and that it provides sufficient support without restricting movement more than necessary. Due to the individual parameters and needs of each patient, such as the anatomical shape of the ankle and foot, weight, height, walking parameters, type of pathology, it is necessary to make the ankle and foot orthoses tailored to the specific patient. Furthermore, the ankle and foot orthosis must withstand cyclical stress, wherein it is necessary to provide sufficient rigidity and firmness. Therefore, when designing and producing the ankle and foot orthosis, it is necessary to individualise the rigidity according to the parameters and needs of the patient, while ensuring comfortable and safe movement for the patient. It is also necessary to shorten and streamline the method of production of such an orthosis as much as possible to ensure that the patient receives a suitable orthosis as soon as possible, which is particularly important for more serious diagnoses that, for example, make it impossible for the patient to walk without such an orthosis. Another important parameter is the weight and size of the orthosis, wherein it is desirable for patient comfort and material saving to provide the lightest possible orthosis with the smallest possible volume. Last but not least, there is a requirement to reduce the cost of production of such an orthosis, because foot and ankle orthoses are usually used for a short period of time, as the patient's need to wear the orthosis ceases or the patient's needs and parameters change when wearing the orthosis for a longer period of time, wherein the parameters of the orthosis need to be adjusted at the same time.

To increase the comfort of the ankle and foot orthosis, an inner lining of soft material is often used to soften the contact surface of the foot and ankle with the orthosis. However, the use of this lining does not result in sufficient softening in critical parts of the orthosis, such as regions of ankles and other points where bones protrude and region of instep, which would also provide sufficient fixation and support to the foot and ankle. Furthermore, the inner lining of soft material and the orthosis itself form two separate components, wherein the connection of these two components must also be resolved, which can lead to complications in use of the orthosis and complicate the production method.

The document US6517505 B1 discloses a one-piece ankle and foot orthosis that is made of a single flexible piece, wherein it is flexible in both the bottom part beneath the foot and in the top part on the medial and lateral sides which encircle the anterior part of the foot. The flexibility of the orthosis contributes to the ease of application, but in some cases it may not provide the necessary ankle and foot support that the patient needs, for example, for proper healing or treatment of walking pathology. Further, for the proper functioning of such an orthosis, the orthosis design is required to extend over the anterior part of the foot, wherein in order to put on such an orthosis it is necessary to force the orthosis open and insert the foot, which is allowed by the flexibility of the design, but when repeated several times, it may lead to wear or damage to the orthosis and loss of the orthosis properties that are required for proper functioning thereof.

The document US6517505 B1 discloses a production method in which a set of physical models of the ankle and foot is first created. Next, the heated formable plastic material is wrapped around the model of the ankle and foot. For this production method, it is necessary to create a number of physical models of the ankle and foot and select the appropriate model based on the physical measurements of the patient's foot and ankle during the production. The orthosis can be optimised by selecting the appropriate thickness of the base material and its type, but this selection is common to all parts of the orthosis, and it cannot be further individualised beyond its shape. The formable plastic piece forming the body of the orthosis encircling the ankle and foot has an almost uniform wall width, wherein it is not possible to further individualise such an orthosis beyond its shape.

The document US20180042751 discloses an ankle and foot orthosis comprising a bottom part adapted to support the plantar region of the foot, that is, providing a tread surface for the foot, where the bottom part is connected to a tibial piece via a "U"-shaped member that encircles the bottom part at the heel and is connected to the tibial piece by a hinge mechanism. Straps are also attached to the bottom part for fixing around the anterior part of the foot, fastening the orthosis to the foot. This solution of the orthosis does not provide sufficient support in the anterior region of the foot and ankle, which can be a major shortcoming in the treatment of some injuries, diseases, or walking pathologies. When walking, the only support on the dorsum of the foot are the straps, which can rub the foot at the points of contact, and the tibial piece, which allows fixation of the ankle joint. The "U"-shaped member is not in contact with the foot, and it provides no additional support. The orthosis is composed of several pieces, which increases the risk of damage of the connection of these pieces during longer use. The document mentions 3D printing for the production of some parts.

It would therefore be useful to provide a solution for an ankle and foot orthosis which would have sufficient rigidity to provide sufficient support for the foot and ankle while ensuring comfortable and safe movement for the wearer, and which would be simple in design and easy to produce. Furthermore, it would be useful to provide a method of production of the foot and ankle orthosis which would take into account parameters and needs of the wearer and reflect them as best as possible in the specific orthosis design, which would be quick and inexpensive and which would optimise the weight of the orthosis as best as possible.

### Summary of the Invention

The above shortcomings are eliminated by a 3D printed ankle and foot orthosis comprising a 3D printed shell, wherein the shell comprises a bottom part and a top part, wherein the top part forms a single piece with the bottom part and the top part comprises a medial part and a lateral part, the bottom part is adapted to support the plantar region of the foot, wherein the medial part is shaped for contact with the medial side of the ankle and foot and extends above the medial ankle region, and the lateral part is shaped for contact with the lateral side of the ankle and foot and extends above the lateral ankle region. The bottom part comprises a rigid region and at least one of the medial part and the lateral part comprises a flexible region. The 3D printed ankle and foot orthosis further comprises an anterior piece located between the medial part and the lateral part, wherein the anterior piece is adapted to encircle the anterior region of the foot and ankle between the medial part and the lateral part. Further, the anterior piece is made of a single material, wherein the medial side of the anterior piece and the lateral side of the anterior piece each comprise at least one transverse cutout from the margin of the medial side of the anterior piece and the lateral side of the anterior piece toward the central part of the anterior piece, wherein the transverse cutouts are adapted to increase flexibility of the central part of the anterior piece at a point between the transverse cutouts.

This design of the orthosis provides sufficient support for the patient's ankle and foot, comfortable and safe movement when using the orthosis, and it is also easy to put on. This is achieved by including the anterior piece in combination with the flexible region in the top part of the shell, which together ensure sufficient encircling of the entire circumference of the foot. The flexible region ensures the bendability of a certain segment of the top part of the shell. This allows the shell to be partially opened and provide access for the patient's foot and ankle, wherein the gap between the medial and lateral parts also helps to make the orthosis accessible. Once the foot and ankle are inserted, the flexible region then allows this segment of the top part of the shell to encircle the ankle and foot in the corresponding region. The anterior piece then provides the encircling of the foot and ankle in the foot and ankle region between the lateral and medial parts, wherein sufficient encircling of the foot and ankle is provided around the entire circumference. The greater rigidity of the rigid region of the bottom part of the shell provides the necessary resistance of the orthosis in the regions where it is most stressed.

The anterior piece preferably comprises a longitudinal central part, a medial side of the anterior piece and a lateral side of the anterior piece, wherein the central part comprises a rigid region and both the lateral side of the anterior piece and the medial side of the anterior piece comprise a flexible region. Furthermore, the wall thickness of the anterior piece is preferably greater in the central part of the anterior piece than on the medial side and lateral side of the anterior piece. The wall thickness of the anterior piece in the central part of the anterior piece varies in the longitudinal direction. In these preferred embodiments, the anterior piece has the necessary rigidity and at the same time a more comfortable movement when using the orthosis is ensured while saving material to produce the orthosis.

The difference in rigidity between the flexible region and the rigid region is preferably provided by one of these options:
- the flexible region comprises a plurality of openings, wherein the flexible region comprises a greater proportion of the volume representation of the openings relative to the volume representation of the 3D printed structure filling the space between the shaped openings than the rigid region,
- the wall thickness of the flexible region at its thinnest point is less than the wall thickness of the rigid region at its thinnest point,
- the flexible region is formed by one wall while the rigid region comprises at least two interconnected walls, or
- the rigid region comprises reinforcing projections.

Preferably, the medial part of the top part of the shell and the medial side of the anterior piece partially overlap and the lateral part of the top part of the shell and the lateral side of the anterior piece partially overlap. This provides a firm encircling of the entire circumference of the foot and ankle, while minimising contact between the edges of the top part of the shell and the anterior piece and the patient's foot and ankle.

In certain types of orthosis, it is preferable if the orthosis comprises a tibial piece at least partially encircling the tibia, wherein the anterior piece at least partially encircles the tibia.

Preferably, the top part of the shell comprises a flexible region in at least one of: the lateral ankle region, the medial ankle region, and the instep region.

The top part of the shell further preferably comprises a posterior part located between the medial part and the lateral part adapted to encircle the posterior region of the foot and ankle. In some types of orthoses, it is preferable to make the orthosis firmer in the posterior region.

The top part of the shell in the posterior region above the region of the heel comprises a gap dividing the medial part and the lateral part. The gap also contributes to the bendability of the medial and lateral sides, making the orthosis easier to access for the foot and ankle.

In a preferred embodiment, the orthosis of the invention includes a clamping mechanism for tightening the anterior piece over the anterior region of the foot and ankle using the medial part and/or lateral part. The shell is preferably connected to the anterior piece via the clamping mechanism.

In some types of orthoses, the bottom part of the shell preferably includes a forefoot surface adapted to support the forefoot of the foot from the level of the metatarsal heads towards the toe of the foot, wherein the forefoot surface has a variable thickness decreasing towards the toe of the foot.

Preferably, a method of production of the 3D printed ankle and foot orthosis is used comprising the following steps:
1) a step of obtaining a 3D model based on the anatomical shape of the patient's ankle and foot,
2) a step of spatially establishing the 3D model based on the anatomical shape of the patient's ankle and foot, either automatically or based on user input by marking at least one point from a plurality of points of the 3D model based on the anatomical shape of the patient's ankle and foot,
3) a step of configuring the orthosis, comprising specifying the design embodiment of the orthosis by entering input parameters via a user interface,
4) a step of designing a 3D model of the orthosis on the basis of the 3D model based on the anatomical shape of the ankle and foot established in space and on the input parameters,
5) a step of sending the data of the 3D model of the orthosis to a 3D printer,
6) a step of making the orthosis by 3D printing,
where the input parameters comprise dimensions of the patient's foot comprising the distance in the direction of the longitudinal axis of the foot between the outermost point of the heel of the foot and the metatarsal head of the little toe, the distance in the direction of the longitudinal axis of the foot between the outermost point of the heel of the foot and the metatarsal head of the big toe, and the width of the foot at a point between the metatarsal head of the little toe and the metatarsal head of the big toe, wherein in the step of designing the 3D model of the orthosis, the dimensions of the shell and the anterior piece are determined based on the length of the foot, the distance in the direction of the longitudinal axis of the foot between the outermost point of the heel of the foot and the metatarsal head of the little toe, the distance in the direction of the longitudinal axis of the foot between the outermost point of the heel of the foot and the metatarsal head of the big toe, and the width of the foot at the point between the metatarsal head of the little toe and the metatarsal head of the big toe, and the 3D model based on the anatomical shape of the patient's ankle and foot.

Preferably, the width of the foot at the point between the metatarsal head of the little toe and the metatarsal head of the big toe is measured with the foot loaded, the distance in the direction of the longitudinal axis of the foot between the outermost point of the heel of the foot and the metatarsal head of the little toe is measured based on the flexion of the little toe, and the distance in the direction of the longitudinal axis of the foot between the outermost point of the heel of the foot and the metatarsal head of the big toe is measured based on the flexion of the big toe.

In the step of designing the 3D model of the orthosis, the distance between the medial part and the lateral part at the level of the metatarsal heads is preferably determined based on the width of the foot at the point between the metatarsal head of the little toe and the metatarsal head of the big toe, the length of the lateral part is determined based on the distance in the direction of the longitudinal axis of the foot between the outermost point of the heel of the foot and the metatarsal head of the little toe, and the length of the medial part is determined based on the distance in the direction of the longitudinal axis of the foot between the outermost point of the heel of the foot and the metatarsal head of the big toe.

In a preferred embodiment, in the step of designing the 3D model of the orthosis, the length of the bottom part of the shell is determined based on the length of the patient's foot or based on the distance in the direction of the longitudinal axis of the foot between the outermost point of the heel of the foot and the metatarsal head of the little toe and the distance in the direction of the longitudinal axis of the foot between the outermost point of the heel of the foot and the metatarsal head of the big toe.

Preferably, the step of configuring the orthosis comprises selecting the type of orthosis comprising at least two types of orthoses from the following group: an ankle and foot orthosis, an ankle, foot, and the back side of the tibia orthosis, and an ankle, foot, and the front side of the tibia orthosis, wherein based on the selected type of the orthosis, the height of the orthosis is determined.

The location of the front edge of the anterior piece is preferably determined based on the level of the metatarsal heads, the width of the front edge of the anterior piece is based on the circumference of the foot at the level of the metatarsal heads, the length of the anterior piece is determined based on the type of the orthosis, and the width of the upper edge of the anterior piece is based on the circumference of the patient's lower extremity at the level of the upper edge of the anterior piece, wherein the anterior piece encircles 20% to 60% of the length of the circumference of the foot, ankle, or tibia.

Preferably, the step of obtaining the 3D model based on the anatomical shape of the ankle and foot of the patient is used, comprising a step of obtaining a 3D scan of the ankle and foot and a subsequent step of modifying the 3D scan of the ankle and foot.

The step of obtaining the 3D model based on the anatomical shape of the ankle and foot of the patient preferably comprises a step of obtaining a 3D scan of the modified physical model of the ankle and foot.

### Description of Drawings

A summary of the invention is further described by means of exemplary embodiments thereof, which are described with reference to the accompanying drawings, in which:
- Fig. 1: shows a 3D printed ankle and foot orthosis according to the first exemplary embodiment of the invention,
- Fig. 2: shows the 3D printed ankle and foot orthosis according to the first exemplary embodiment of the invention from the lateral side,
- Fig. 3: shows the 3D printed ankle and foot orthosis according to the first exemplary embodiment of the invention from the front side,
- Fig. 4: shows the 3D printed ankle and foot orthosis according to the first exemplary embodiment of the invention from the top side,
- Fig. 5: shows the 3D printed ankle and foot orthosis according to the first alternative embodiment of the invention with a tibial piece,
- Fig. 6: shows the 3D printed ankle and foot orthosis according to the second alternative embodiment of the invention with a tibial piece and two anterior pieces,
- Fig. 7: shows a detail of the anterior piece according to the first exemplary embodiment of the invention,
- Fig. 8: shows a transverse section through the anterior piece according to the first exemplary embodiment of the invention,
- Fig. 9: shows a detail of the anterior piece according to an alternative embodiment of the invention,
- Fig. 10: shows a transverse section through the anterior piece according to an alternative embodiment of the invention,
- Fig. 11: shows diagrams of the lower extremity from the lateral side (left) and medial side (right) with anatomical regions marked,
- Fig. 12: shows diagrams of the lower extremity from the back side (left) and bottom side (right) with anatomical regions marked,
- Fig. 13: shows a diagram of the method of production of the 3D printed ankle and foot orthosis.

### Exemplary Embodiments of the Invention

Exemplary embodiments of the invention of the 3D printed orthosis according to the exemplary embodiments will be explained herein with reference to the corresponding drawings.

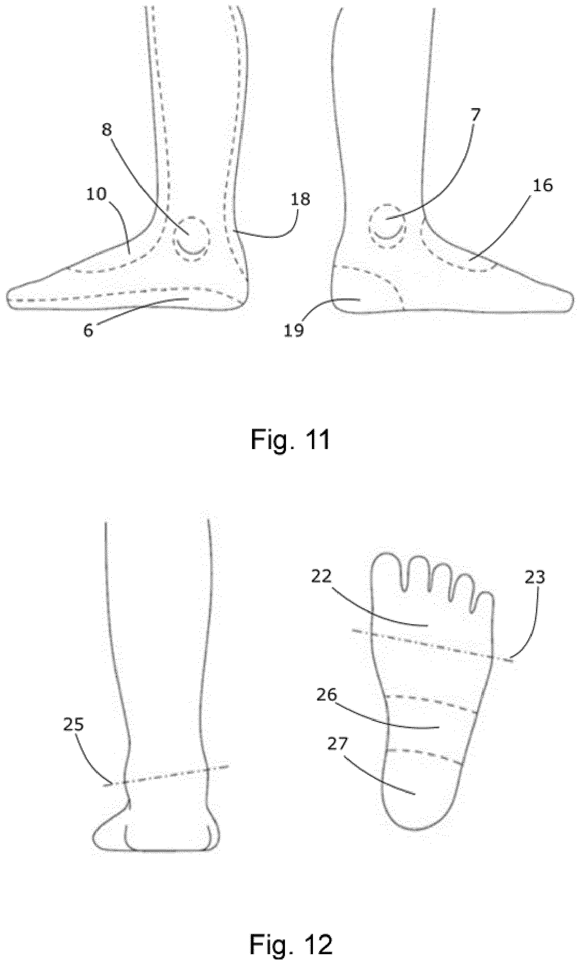

Some terms used in the description of the exemplary embodiments of the invention and their function will be explained herein. The tread surface is the surface with which the patient's foot is in contact when the patient's foot treads and via which the patient's weight is transferred to the outer surface or orthosis. The lateral ankle region 8 is the region of the projecting outer surface of the ankle, which is caused by the presence of the lateral ankle or the projection of the fibula. The medial ankle region 7 is the region of the projecting outer surface of the ankle, which is caused by the presence of the medial ankle or the projection of the tibia. By the level 25 of the ankles is meant the axis passing through the top points of the medial ankle and lateral ankle. By the hindfoot 27 is meant the back part of the foot including the calcaneus and the talus. By the midfoot 26 is meant the middle part of the foot, which includes the navicular bone, the inner cuneiform bone, the middle cuneiform bone, the outer cuneiform bone, and the cuboid bone. By the forefoot 22 is meant the front part of the foot, which includes 5 metatarsal or instep bones and the toes. By the level 23 of the metatarsal heads is meant the axis essentially passing through the metatarsal bone heads. By the big toe metatarsal head is meant the 1st metatarsal or instep bone head. By the little toe metatarsal head is meant the 5th metatarsal or instep bone head. By the region 16 of the instep is meant the region on the upper side of the foot corresponding to the upper side of the metatarsal bones. By the posterior region 18 is meant the region of the back side of the foot, ankle, and tibia from the heel to the top side of the tibia. By the anterior region 10 is meant the region of the dorsum of the foot and the front side of the ankle and tibia from the region 16 of the instep to the top side of the tibia. By the posterior region 18 or anterior region 10 of the ankle and foot is meant only the ankle and foot part of the posterior region 18 or anterior region 10. The individual anatomical regions are exemplarily shown in the diagrams in Fig. 11 and Fig. 12.

The location of the segments and pieces of the orthosis and the rigid and flexible regions on the orthosis is specified by means of different anatomical regions of the patient's foot and ankle or tibia. The inner walls of the ankle and foot orthosis are based on the anatomical shape of the ankle and foot and possibly the wearer's tibia, wherein the orthosis fits tightly to the ankle and foot over a significant part of the outer surface of the foot and ankle. Claiming that a region is located in an anatomical region of the ankle and foot means that the region of the orthosis in question is located perpendicularly above the outer surface of the foot, ankle, or tibia corresponding to the anatomical region in question. The anatomical regions are specified above, wherein it is obvious to the person skilled in the art of anatomy of the foot and ankle how these regions are defined.

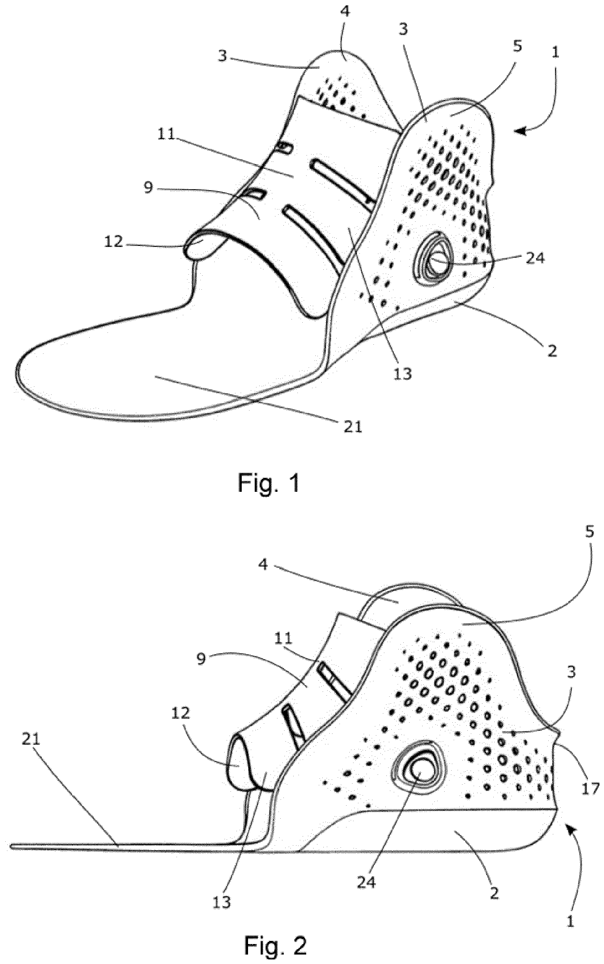

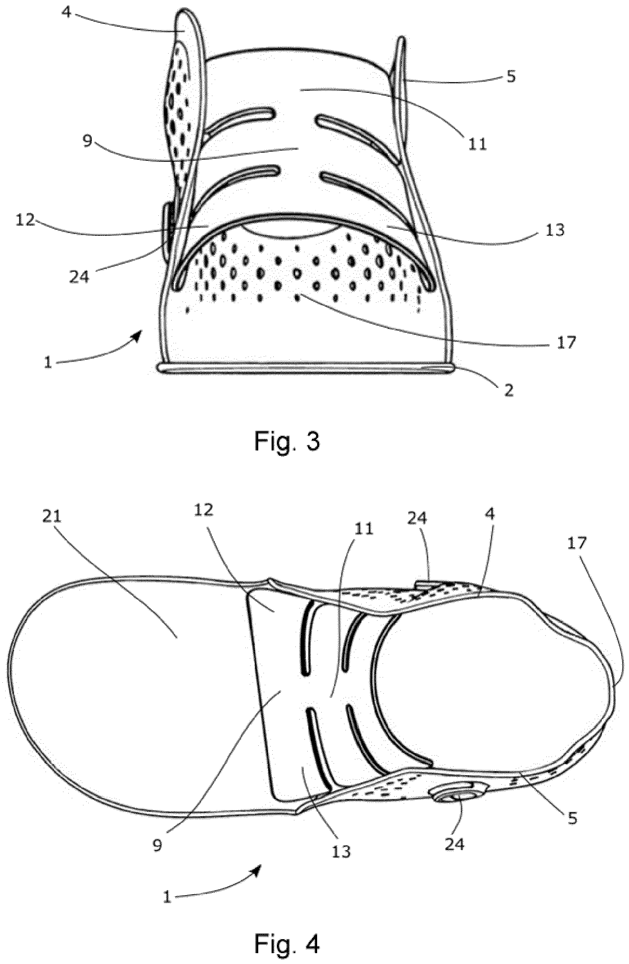

The 3D printed ankle and foot orthosis according to the first exemplary embodiment is shown in Fig. 1, Fig. 2, Fig. 3, and Fig. 4. The 3D printed orthosis according to the first exemplary embodiment comprises a 3D printed shell 1, which forms the main body of the orthosis, and which comprises a bottom part 2 and a top part 3. The bottom part 2 of the shell is adapted to support the plantar region 6 of the foot and extends up to a maximum orthosis height of 20% of the total length of the foot. The bottom part 2 of the shell thus means the part of the shell 1 located in particular under the plantar region 6 of the foot, which by its inner side forms the tread surface for the foot and provides suitable support for the foot from its bottom side and which is adapted by its outer side to contact the outer surface.

The bottom part 2 and top part 3 of the shell together form one 3D printed piece, wherein the distinction between the bottom part 2 and the top part 3 will be explained herein. The height of the bottom part and 2 of the shell corresponds to the height to which the tread surface for the foot of the bottom part 2 of the shell extends. The height to which the tread surface extends depends on how wide the tread surface is in relation to the foot. The foot is spread laterally when treading, wherein when treading on a flat surface, the tread surface is essentially flat, since the tread surface of the flat surface is the part of the flat surface that is in contact with the foot when the foot treads. In case of the orthosis of the invention, however, the shell 1 is adapted to support the foot at least partially from the sides, wherein the shell defines a space for the foot to spread into when treading, and does not allow the foot to spread laterally as it is in the case of treading on a flat surface, and the foot thus partially exerts a force also on the lateral sides of the shell 1 in contact with the foot, wherein the segments of the inner surface of the lateral sides of the shell 1 in contact with the foot also form a tread surface. The bottom part 2 of the shell then extends to a height corresponding to the height of the tread surface at that point of shell 1 and this tread surface extends to a maximum orthosis height of 20% of the total length of the foot, wherein this depends on the design of the orthosis. The height of the tread surface, and therefore the bottom part 2 of the shell, can vary along the length of the foot, wherein it is usually preferable if the bottom part 2 of the shell is higher in the region of the heel than in the region of the toe of the foot.

While the bottom part 2 of the shell is adapted to mediate contact with the external environment and support the plantar region 6 of the foot, the top part 3 of the shell is adapted to fix the foot and ankle and support the rest of the foot and ankle and, in some types of orthoses, even the tibia. The top part 3 of the shell comprises a medial part 4 and lateral part 5, which are adapted in size and shape to contact the lateral sides of the foot and ankle. The top part 3 of the shell connects to the bottom part 2 of the shell at the point where the tread surface of the shell 1 ends, or where the walls of the bottom part 2 of the shell end, which are adapted to support the plantar region 6 of the foot. The top part 3 of the shell extends above the regions 7, 8 of the medial and lateral ankle, approximately 2 cm above the level 25 of the ankles, and completely covers the regions 7, 8 of the medial and lateral ankle and follow their shape, wherein the top part 3 of the shell is adapted to support them.

The bottom part 2 of the shell includes a rigid region that is adapted to a larger load compared to the other parts of the orthosis, and the top part 3 of the shell comprises a flexible region. The flexible regions can have two different purposes, and therefore two different types of flexible regions adapted to that purpose. The first type of the flexible region is a region adapted to soften the contact between the orthosis and the ankle and foot in critical regions of the ankle and foot, such as the regions 7, 8 of the ankles or other points where the bones project to a greater extent, or the sensitive ankle and foot regions of the orthosis wearer. The first type of the flexible region is oriented towards a given critical point and forms an inner wall of the shell 1 adjacent to that point, and partially the inner volume of the shell 1 adjacent to that inner wall. The first type of the flexible region of the shell 1 preferably has a lower rigidity on its inner wall oriented towards the critical region than at points further away from the inner wall. In the position where the orthosis is put on the wearer's foot and ankle, the first type of the flexible region is preferably in constant deformation, thereby providing support to the given point as well as the appropriate level of freedom that is provided by the flexibility of the region. Further, the first type of the flexible region is preferably adapted to how the orthosis is stressed during use, or walking, wherein the dimensions and the degree of rigidity of the first type of the flexible region take into account the entire cycle of stress during walking.

The second type of the flexible region is then adapted to increase the flexibility, or bendability, of certain regions, of the top part 3 of the shell and the anterior piece 9, which ensure the fixation of the orthosis around the ankle and foot of the wearer. In particular, the purpose of the second type of the flexible region is to ensure proper encircling of the orthosis around the foot and ankle of the patient while ensuring an easy method of putting on and taking off the orthosis of the invention. With respect to the shell 1, these flexible regions for a substantial part, approximately by 60% to 100%, flank the margins of the segments of the top part 3 of the shell, which are adapted in terms of their size and shape to contact the medial and lateral sides of the ankle and foot, whereby these flexible regions allow bending of these segments of the top part 3 of the shell relative to the rest of the orthosis. These segments can then be bent to create sufficient space for the insertion of the patient's foot and ankle, and then the foot can be encircled by these segments. In the case of the anterior piece 9, this type of flexible region then provides for the bending of the individual segments of the medial side 12 and the lateral side 13 of the anterior piece so that, after the orthosis has been fitted to the ankle and foot or tibia of the patient, the anterior piece 9 is suitably adapted to the anatomical shape of the foot and ankle or tibia of the patient. The second type of the flexible region runs through the entire cross-section of the wall of the top part 3 of the shell or the anterior piece 9 at the point of bending, and flanks most of the bend band of the segment. In some embodiments, it is possible that the flexible region of the first type adapted to soften a particular region of the ankle and foot will blend with the flexible region of the second type, wherein the flexible region of the first type may also partially contribute to the bendability of a particular segment of the top part 3 of the shell or the anterior part 9 of the shell.

The term flexible region means a three-dimensional elastic region of lower rigidity that deforms to a greater extent than the rigid region when an external force is applied, wherein it returns to its original dimensions when the external force is removed. When a force is applied to the flexible region by an external body, the flexible region exerts a force at the points of contact with the external body corresponding to the rigidity of the flexible region on the external body. The external body in the context of this document is particularly the ankle and foot of the wearer of the orthosis. The rigidity of the given region is further affected by the material used, wherein different materials exhibit different rigidityes, wherein both the material properties of the used printing material and the design of the particular 3D printed structure in the region must be taken into account when determining the appropriate rigidity of the given region. The 3D printed orthosis of the invention is printed from a single material, wherein the different rigidity of the regions of the orthosis is affected only by the design of the 3D printed structure of that region. The difference in rigidity between the flexible and rigid region can be provided in the following ways:
1. the flexible region comprises a larger proportion of the volume representation of the openings relative to the volume representation of the 3D printed structure filling the space between the openings,
2. the wall thickness of the flexible region at its thinnest point is less than the wall thickness of the rigid region at its thinnest point,
3. the flexible region is formed by one wall while the rigid region comprises at least two interconnected walls,
4. the rigid region comprises reinforcing projections.

The first variant of the embodiment of different rigidity of the flexible and rigid region will be explained herein. Depending on the specific shape, distance, and size, the openings adjust the rigidity of the flexible region and give it different directional expansion. The openings are the volumetric cutouts of a specific shape in the volume of the 3D printed shell. The rigidity of the flexible region is determined by specific shape, distance, and size of the openings in the given volume. The openings are distributed in the volume of the shell 1 such that the 3D printed structure filling the volume between them has the structure of a three-dimensional network. In the case where the openings are smaller, have a shape that provides a greater rigidity to the surrounding structure, and/or are at a distance from each other, thicker 3D printed structures are achieved which fill the space between the openings and provide a greater rigidity to the 3D printed structure. By thicker 3D printed structures are meant structures with a larger cross-section at the location between the openings and with a higher volume representation in proportion to the volume representation of the openings. If larger openings have a shape that provides a lower rigidity of the surrounding structure, and/or are located close together, weaker 3D printed structures are achieved which fill the space between the openings, wherein they provide a lower rigidity to the 3D printed structure. By weaker 3D printed structures are meant structures with a smaller cross-section at the point between the openings and with a smaller volume representation relative to the volume representation of the openings.

In preferred embodiments, the volume representation of the openings decreases towards the margins of the flexible region, wherein at the margins of the flexible region the rigidity increases, approaching the rigidity of other regions adjacent to the flexible region. In case of the first type of the flexible region, preferably the rigidity increases from the region of contact with the critical region of the ankle and foot of the wearer, where the rigidity of the 3D printed structure is the smallest, and thus a only small force is needed to deform this structure, to the margins of the flexible region, where the rigidity of the 3D printed structure is the largest, and thus a larger force is needed to deform this structure. It is preferable if the flexible region of the first type gives the ankle and foot some freedom in that region to increase the comfort of the orthosis, but only to a certain extent, as the orthosis must also provide sufficient support in the given point. By the region of contact with the critical region of the wearer's ankle and foot is meant the volume of the flexible region corresponding to the difference between the volume of the flexible region when no external forces are applied and the volume when the orthosis is put on the patient's ankle and foot in the resting position of the foot and ankle. In the region of contact with the critical region of the ankle and foot of the wearer, it is therefore preferable to have less rigid structures which do not exert too much force on the critical region of the ankle and foot and do not irritate or even bruise it unnecessarily. However, with greater forces acting on the flexible region, when the parts of the flexible region farther away from the region of contact with the higher rigidity have already begun to deform, the flexible region begins to show more resistance, thus providing support to the ankle and/or foot. For the first type of the flexible region, this method of providing a lower rigidity of the 3D printed structure is particularly preferable.

The second variant of the embodiment of different rigidity of the flexible and rigid region will be explained herein. The thinner the wall formed by the 3D printed structure, the more prone it is to deformation. In case of the embodiment of the first type of the flexible region, the second variant is particularly preferable when combined with the use of thermoplastic elastomers for 3D printing of the orthosis, such as thermoplastic polyamide (TPA), thermoplastic polyurethane (TPU), or copolymer blend (TPE). When using these materials, the overall design of the 3D printed piece is essentially flexible, wherein higher rigidity is only achieved for walls with a greater thickness. The rigid region thus has a thicker wall in this variant with essentially 100% of the volume filled with 3D printed structure. The lower rigidity of the flexible region is then provided by reducing the wall thickness. Similarly, as described for the first variant of the embodiment of different rigidity, it is preferable when the wall thickness of the first type of the flexible region decreases from the region of contact with the critical region of the ankle and foot of the wearer to the margins of the flexible region. The flexibility of the flexible region is also affected by the size of the surface occupied by the flexible region and by how the wall thickness varies from the centre of the flexible region to its margins. The wall thickness, the size of the flexible region, and the degree of variability of the wall thickness in the direction from the region of contact with the critical region of the ankle and foot to the margins of the flexible region then determines the overall rigidity of the region, i.e., how much resistance the flexible region will exert, or how much force the flexible region will exert on the ankle and foot at different degrees of deformation. In case of the embodiment of the second type of the flexible region, the bendability of the required segments of the top part 3 of the shell, which are adapted in size and shape to encircle the ankle and foot, and of the anterior piece 9 is provided by reduced wall thickness in the region flanking the margin of the segment.

The third variant of the embodiment of different rigidity of the flexible and rigid region will be explained herein. In this variant of providing different flexibilities of the regions, the flexible region is formed by one wall while the rigid region comprises at least two interconnected walls. In some embodiments, it may be preferable to provide the rigidity of the rigid region by two interconnected walls, wherein they may comprise multiple interconnected walls. These walls then provide support to each other and are thus less prone to deformation. The flexible regions can then be made up of only one wall, providing increased flexibility, which is preferable when using thermoplastic elastomers as a material for 3D printing the orthosis. The fourth variant of the embodiment of different rigidity of the flexible and rigid region will be explained herein. Reinforcing projections (e.g., ribs, beams) placed longitudinally, transversely, or diagonally in the rigid region increase the rigidity of the wall on which they are placed by changing the variable cross-section of the wall. For example, if longitudinal parallel ribs are placed on a wall, the wall is significantly reinforced against bending around an axis perpendicular to the longitudinal direction of the ribs. Placing a raised grid structure on the wall would then reinforce the wall around an axis perpendicular and parallel to the longitudinal direction of the ribs.

It is possible to combine the individual variants of providing different rigidity of the rigid and flexible regions, wherein the rigidity of the rigid region can be increased by one or a combination of variants and, at the same time, the rigidity of the flexible region can be provided by other variants or a combination of variants of providing different rigidity of the regions.

The medial part 4 and lateral part 5 are not adapted to encircle the entire anterior region 10 of the foot and ankle, wherein the orthosis further comprises the anterior piece 9 in the shape of a fan-shaped pelota adapted in shape and size to encircle the anterior region 10 of the foot and ankle between the medial and lateral parts. The anterior piece 9 is a separate 3D printed piece. The anterior piece 9 comprises a longitudinal central part 11 which forms the backbone of the entire anterior piece 9, a lateral side 13 of the anterior piece, and a medial side 12 of the anterior piece. The longitudinal central part 11 of the anterior piece is located in the anterior region 10 of the foot and ankle, the medial side 12 of the anterior piece is connected to the central part 11 and encircles the foot and ankle in the anterior region 10 on the medial side of the foot and ankle away from the central part 11 of the anterior piece, and the lateral side 13 of the anterior piece is connected to the central part 11 of the anterior piece and encircles the foot and ankle in the anterior region 10 on the lateral side of the foot and ankle away from the central part 11 of the anterior piece. The anterior piece 9 by its design extends by the medial side 12 between the foot and ankle and the medial part 4 and by its lateral side 13 between the foot and ankle and the lateral part 5.

The central part 11 of the anterior piece comprises a rigid region, and the medial side 12 and lateral side 13 of the anterior piece each comprise a flexible region. In the first exemplary embodiment, the thickness of the anterior piece 9 decreases from the central part 11 in both directions to the edge of the medial side 12 of the anterior piece and the edge of the lateral side 13 of the anterior piece. Minimising the thickness of the anterior piece 9 at the margins of the medial side 12 and lateral side 13 of the anterior piece facilitates a smoother transition between the anterior piece 9 and the top part 3 of the shell, thereby reducing the impact of this transition on the patient's foot and ankle. The difference in flexibility is provided by using the second variant, or by using different thickness. The bendability of each segment of the anterior piece 9 relative to the central part 11 increases with distance from the longitudinal central part 11 of the anterior piece. The anterior piece 9 further comprises cutouts 14 from the margin of the medial side 12 and the lateral side 13 of the anterior piece toward the central part 11, where the cutouts 14 are transversely oriented relative to the longitudinal axis of the anterior piece 9. These cutouts 14 increase the flexibility of the medial side 12 and lateral side 13 of the anterior piece by dividing them into smaller, separate segments, while also providing a greater flexibility of the central part 11 of the anterior piece at the point between the cutouts 14.

The anterior piece 9 of the first exemplary embodiment comprises two transverse cutouts 14 on the medial side 12 of the anterior piece and two transverse cutouts 14 on the lateral side 13 of the anterior piece, wherein each pair of cutouts 14 is located at the same level and extends to the central part 11 of the anterior piece. The individual segments of the medial and lateral sides 12,13 of the anterior piece divided by the cutouts 14 are connected only by the central part 11, wherein the central part 11 is more prone to bending at these connections, or at the points where these cutouts 14 meet, than at the points outside the cutouts 14. Thus, the anterior piece 9 comprises 3 mutually movable segments (bottom, middle, upper) divided by corresponding cutouts 14 and connected only by the central part 11 of the anterior piece. Thus, the shape adaptability of the anterior piece 9 is provided by a combination of the first variant, or by openings, since the cutouts 14 can be considered openings, and the second variant, or by a variable wall thickness between the central part 11 and the lateral and medial sides 12,13 of the anterior piece. The front edge of the anterior piece 9 is located at the level 23 of the metatarsal heads, wherein it substantially encircles the anterior part of the circumference of the foot in a plane corresponding to the level 23 of the metatarsal heads. The upper edge of the anterior piece 9 is located at the level 25 of the ankles, wherein it substantially encircles the anterior part of the ankle circumference in a plane corresponding to the level 25 of the ankles. The anterior piece 9 overlaps on both sides with the medial and lateral parts 4, 5 of the top part 3 of the shell by at least 1 cm of its width.

The top part 3 of the shell further extends only approximately to the level 23 of the metatarsal heads of the foot, where the medial part 4 extends to the level of the metatarsal head of the big toe and the lateral part 5 extends to the level of the metatarsal head of the little toe. The height of the medial part 4 and the lateral part 5 gradually increases in the direction of the longitudinal axis of the foot from the posterior region 18 to the highest point above the regions 7, 8 of the ankles and then gradually decreases to the level 23 of the metatarsal heads, where their front ends connect to the bottom part 2 of the shell. The top part 3 of the shell of the first exemplary embodiment comprises a clamping mechanism 20 comprising two integrated grips 24, a fastening ring, and a hook-and-loop strap. The medial part 4 comprises one integrated grip 24 adapted to fix the fastening ring. The lateral part 5 comprises a second integrated grip 24 adapted to fix the hook-and-loop strap, wherein the hook-and-loop strap is adapted to be threaded through the fastening ring and locked by hook-and-loop. The clamping mechanism 20 is adapted to encircle the anterior region 10 through the anterior piece 9, thereby firmly fixing the orthosis of the first exemplary embodiment to the ankle and foot. The location of the fastening ring and the hook-and-loop strap can be reversed. Further, preferably, the anterior piece 9 is connected to the clamping mechanism 20. In the first exemplary embodiment, the anterior piece 9 is releasably connected to the hook-and-loop strap of the clamping mechanism 20 by hook-and-loop mechanism, wherein the anterior piece 9 comprises one hook-and-loop part on its upper side and the hook-and-loop strap comprises a second hook-and-loop part on its bottom side. The top part 3 of the shell comprises a marginal curve approximately 1 cm wide around its circumference in the direction from the margin of the top part 3 of the shell. Within the marginal curve, the wall thickness of the top part 3 of the shell decreases towards the margin of the top part 3 of the shell.

In the first exemplary embodiment of the orthosis of the invention, the top part 3 of the shell includes two flexible regions of the first type in the medial ankle region 7 on the medial part 4 and the lateral ankle region 8 on the lateral part 5. Both flexible regions are made based on the first variant to reduce the rigidity of the region by using openings in the volume of the shell, wherein the volume representation of the openings is reduced from the centres of the flexible regions to their margins. In the posterior region 18, the medial part 4 and the lateral part 5 are connected, wherein together they encircle the foot in the region of the heel also partially from the back side. In the posterior region 18 above the level of the heel, the medial part 4 and the lateral part 4 subsequently diverge to form a gap therebetween oriented parallel to the posterior region 18. This gap allows the segments of the medial part 4 and the lateral part 5 to bend slightly away from each other even in the absence of the flexible regions of the second type, but damage or wear of the orthosis may occur. The flexible region of the second type and this gap together contribute to the correct bendability of the medial and lateral parts 4, 5 of the top part 3 of the shell of the first exemplary embodiment. After securing the foot and the ankle in the orthosis, it is thus possible to bring the medial and lateral parts 4, 5 slightly closer together by means of the clamping mechanism 20, thereby fixing the ankle and foot from the lateral sides and from the back side, wherein by means of the anterior piece 9 and by tightening the clamping mechanism 20, also the anterior region 10 of the foot and ankle is firmly fixed.

The bottom part 2 includes a rigid region that is adapted to greater loads compared to the other parts of the orthosis. The size, location, and specific rigidity of the rigid region depends on the individual parameters and needs of the specific patient for whom the orthosis is intended. The bottom part 2 of the first exemplary embodiment comprises a rigid region at the point corresponding to the region of the hindfoot 27 in the vicinity of the heel, a region of the outer part of the midfoot 26, and a region of the middle part of the forefoot 22, wherein in particular these regions of the orthosis are commonly subjected to the greatest load during use of the orthosis. The higher rigidity of the rigid region of the bottom part 2 of the shell is achieved by filling 100% of the volume of the rigid region with the 3D printed structure. Behind the level 23 of the metatarsal heads in the direction of the longitudinal axis of the foot towards the toe, the orthosis is formed only by the bottom part 2 of the shell, which in this region is formed only by the forefoot surface 21 adapted to support the forefoot 22 of the foot. The forefoot surface 21 approximately follows the shape of the foot in the region of the forefoot 22, wherein it is sized to be approximately 1 to 2 cm longer than the foot.

The forefoot surface 21 of the first exemplary embodiment has a variable thickness decreasing towards the toe of the foot. In some cases, it is preferable for the orthosis to assist the toe of the foot in rebounding, which is achieved by providing an appropriate degree of elasticity of the forefoot surface 21, which is achieved by adjusting the wall thickness of the forefoot surface 21 or its rigidity corresponding to the parameters and needs of the wearer. The forefoot surface 21 may therefore be considered as a flexible region of the third type, the purpose of which is to support the forefoot 22 during walking by suitably adjusting its thickness, and thus by a second variant of the design of different rigidity. The thickness of the forefoot surface 21 preferably decreases towards the toe of the foot. If the orthosis does not need to provide support in the region 22 of the forefoot of the foot, it is preferable not to comprise the forefoot surface 21 in the design of the orthosis. If maximum foot support is required, it is then preferable to provide maximum rigidity of the forefoot surface, wherein the forefoot surface 21 preferably comprises a rigid region and does not comprise a variable thickness. Often, however, the rigidity of the forefoot surface 21 is preferably somewhere in between these cases, wherein it is preferable for the thickness of the forefoot surface 21 to decrease towards the toe, thereby achieving comfortable walking while providing necessary support to match the wall thickness or rigidity.

When the orthosis of the invention is fastened to the foot and ankle of the patient by the clamping mechanism 20, the medial and lateral parts 4, 5 of the top part 3 of the shell are brought closer to the ankle and foot of the patient by means of the flexible regions of the second type located on the medial and lateral parts 4, 5 of the shell. The shell 1 of the orthosis together with the anterior piece 9 encircle the foot around the entire circumference, providing sufficient support, comfortable and safe movement and at the same time allowing easier removing and putting on of the orthosis.

Alternative embodiments and other exemplary embodiments of the 3D printed orthosis of the invention will be explained herein.

In further exemplary embodiments, the medial part 4 and/or the lateral part 5 may be adapted to partially encircle the anterior region 10 of the foot, wherein when encircled there is a certain gap therebetween in the anterior region 10 of the foot. In relation to the length of the circumference along which the individual segments of the medial and lateral parts 4, 5 are adapted to encircle the ankle and foot, their bendability is adjusted using the second type of the flexible regions. Preferably, flexibility is provided by the flexible regions of the second type of the medial part and lateral part 4, 5 such that the top part 3 of the shell can be opened to a degree sufficient for comfortable insertion of the foot without the need to apply brute force or risking damage to the shell 1. Between the medial part 4 and the lateral part 5, there is the anterior piece 9, which encircles the foot, ankle, or tibia, at a point between the medial part 4 and the lateral part 5, wherein it again preferably overlaps when encircling the foot, ankle, or tibia with the lateral part 5 and the medial part 4 of the top part 3 of the shell. Preferably the lateral part 5 and the medial part 4 overlap with the anterior piece 9, and the margins of the lateral part 5 and the medial part 4 are thus not in contact with the lower extremity of the patient, wherein the margins of the anterior piece 9 can be better adapted to contact the lower extremity of the patient, or they can be softened and thinned, as they are not subject to high demands on rigidity, and in addition, the anterior piece 9 can be dimensionally adapted such that its margins are not at critical points of the foot, ankle, or tibia, for example in the region 16 of the instep.

In another alternative embodiment, the anterior piece 9 overlaps with the medial part 4 and lateral part 5, wherein the sides of the medial part 4 and lateral part 5 oriented toward the anterior region 10 of the patient's lower extremity are partially located between the patient's extremity and the anterior piece 9. In further embodiments, the anterior piece 9 is located on one side between the top part 3 of the shell and the lower extremity of the patient and on its other side is located above the top part 3 of the shell. There are various combinations of these embodiments, which vary depending on the needs and parameters of the patient, and the appropriate type of the orthosis.

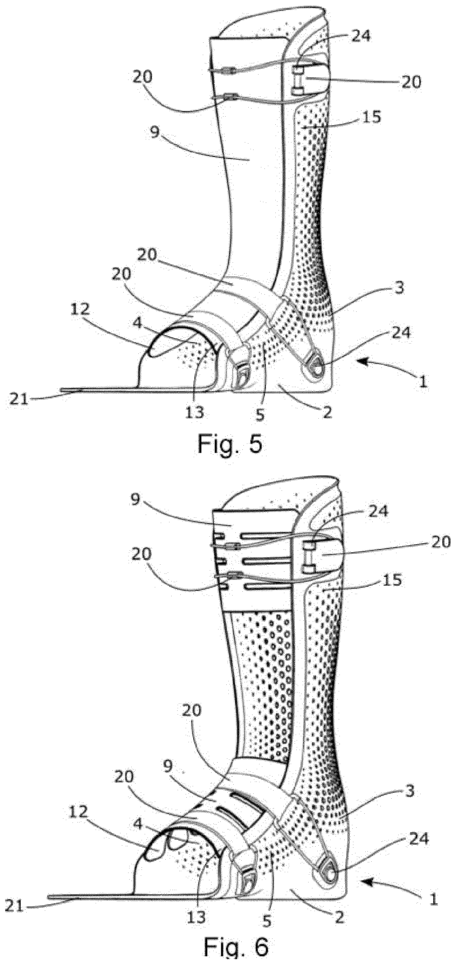

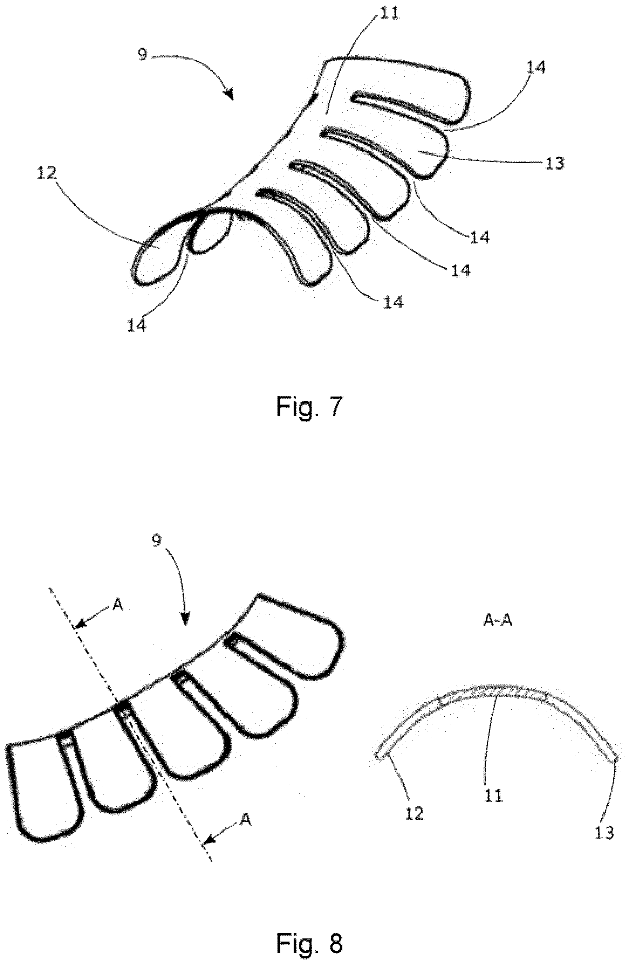

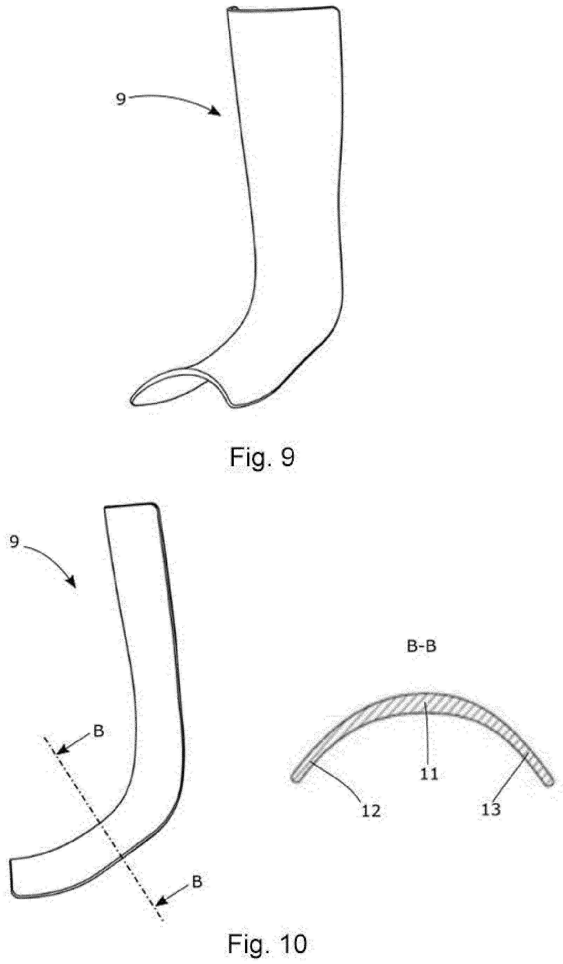

The anterior piece 9 can be further provided in various ways. The anterior piece 9 may, for example, include various embodiments of the cutouts 14 which will be adapted depending on the support and support points to be provided to the ankle and foot. The cutouts 14 may be considered as a flexible region of the first variant of the embodiment of different rigidity, since the cutouts 14 may be considered openings, wherein the cutouts 14 may be replaced by another variant of the embodiment of different rigidity. The anterior piece 9 of the first exemplary embodiment is shown in Fig. 7 and Fig. 8. For example, in the case of thermoplastic elastomers as a material for 3D printing the anterior piece 9, it is preferable to replace these cutouts 14 with thin walls connecting the individual thicker segments of the medial side 12 and the lateral side 13 of the anterior piece to allow them to bend together. The anterior piece 9 does not have to comprise the cutouts 14 or other flexible regions providing bending of the individual segments of the anterior piece 9 at all, in certain cases where it is necessary to provide the highest possible fixation of the foot and ankle. Such an embodiment is shown in Fig. 5, Fig. 9, and Fig. 10, where the anterior piece 9 does not include the cutouts 14 and has only thinned walls at the medial and lateral margins of the anterior piece 9. In alternative embodiments, the anterior piece 9 comprises a flexible region of the first type.

The longitudinal central part 11 of the anterior piece may further include a variable thickness in the longitudinal direction, and the anterior piece 9 may thus have a different flexibility in different regions in bending about the transverse axis of the anterior piece 9 at a given point. This is particularly preferable in higher orthosis embodiments. In some orthoses, it may be preferable for the thickness of the central part 11 in the region 16 of the instep and the region of the tibia to be greater than the thickness of the central part 11 in the region of the anterior bending arch, which will provide firm encircling of the region 16 of the instep and tibia while allowing for smooth bending of the foot at the ankle during walking. In other orthoses, it may be preferable to achieve a change of the bending rigidity of the anterior piece 9 by adjusting the thickness of the central part 11 in the region of the anterior bending arch and in the region 16 of the instep. In this way, it is possible to achieve a specific rigidity of the anterior piece 9 in the region of the anterior bending arch, whereby the anterior piece 9 produces a precisely set resistance to foot bending at the ankle during walking which should be provided by the anterior piece 9 of the orthosis based on the clinical requirement, while at the same time it is not greater than is absolutely necessary.

In further embodiments, the shell 1 may comprise also a posterior part 17 that provides support and covers the foot and ankle in the posterior region 18. This embodiment preferably comprises the flexible region of the second type that helps to bend the medial and lateral part 4, 5 away from each other, or to open the orthosis. In order to save material and provide a venting solution, an embodiment is also preferable where the posterior part 17 comprises a hole, the posterior part 17 thus in particular in effect forms a connection between the lateral part 5 and the medial part 4 above the level 25 of the ankles, wherein it is preferable when the posterior part 17 in the region of this connection comprises a flexible region of the first and/or second type.

In alternative embodiments, the shell 1 may include holes at certain points. In some embodiments, it is preferable when the orthosis comprises an opening in the region 7, 8 of the ankle, the ankle may go through this hole, wherein it is not necessary to address a flexible region of the first type for the ankle. In some embodiments, it is preferable when the shell 1 comprises a hole in the base region of the fifth metatarsal and/or in the hindfoot 27 under the heel, wherein contact of these regions of the foot with the external environment is provided. This is mainly due to the need to ensure a suitable response of the external environment to the foot. A rigid region is preferably located around the hole region in the bottom part 2 of the shell, which compensates for the loss of rigidity caused by the hole. In some embodiments, the shell 1 may comprise a hole in the region of the navicular bone, in cases where the orthosis is not required to provide support in that region. The advantage of implementing holes is to save materials and provide ventilation for the orthosis. To ensure adequate ventilation, it is also preferable to comprise several smaller holes in the regions where ventilation is required. The ventilation holes are preferably small and at a greater distance from each other, wherein they affect the rigidity of the design of the shell 1 as little as possible. In some embodiments, the ventilation may also be provided by flexible regions of the first variant of different rigidity.

In further alternative embodiments, the orthosis of the invention may further include a tibial piece 15 adapted to at least partial encircle, or fixate, the tibia. The tibial piece 15 may be rotatably movably connected to the top part 3 of the shell. In this embodiment, the tibial piece 15 may for example be connected by a hinge mechanism, wherein its one side is connected via the first hinge mechanism to the lateral part 5 and its other side is connected via the second hinge mechanism to the medial part 4. In another embodiment, the tibial piece 15 may be connected to the top part 3 of the shell via only one hinge mechanism on one side. The hinge mechanism can be attached to the individual parts of the orthosis, for example by screws drilled through the shell or by a glued connection. In a further embodiment, the connection of the tibial piece 15 and the top part 3 of the shell may be made by an elastic member, such as a rubber piece, which does not have a firm rotational axis and thus allows movement in different directions. In another embodiment, the tibial piece 15 may be a firm component of the top part 3 of the shell, wherein the medial part 4 and lateral part 5 extend above the level 25 of the ankles and smoothly transition into the tibial piece 15, which partially encircles the tibia. In this embodiment, the tibial piece 15 can be considered a component of the top part 3 of the shell, wherein the tibial piece is formed by the medial part 4, the lateral part 5, or the posterior part 17 of the top part 3 of the shell. This type of orthosis is shown in Fig. 5 and Fig. 6. In these embodiments, the tibial piece 15 encircles the tibia mainly from the back side, or encircles the calf, and there is a gap along the entire front side of the tibial piece 15 that allows the orthosis to be put on comfortably from the front side, wherein it is an orthosis for the ankle, foot, and the back side of the tibia. In this embodiment of the orthosis, the anterior piece 9 also at least partially encircles the anterior region 10 at the level of the tibia. Here, it is preferable if the tibial piece 15 along its back side comprises a flexible region of the second type that allows it to be expanded to the sides. In another embodiment, the tibial piece 15 may be adapted to fix the tibia particularly from the front side and from the sides, wherein the orthosis is thus adapted to be put on from the back side by moving the foot from the back side of the tibial piece 15 into the space between the medial and lateral parts 4, 5. In this case, it is an orthosis of the ankle, foot, and front side of the tibia orthosis type. Similarly, it is again preferable when the tibial piece 15 comprises along the front side a flexible region of the first type for the orthosis to be easier to put on.

The embodiment of the anterior piece 9 may also vary depending on the type of the orthosis. In the case of the ankle, foot, and back side of the tibia orthosis, the anterior piece 9 may also be adapted to encircle the tibia up to the region below the knee joint. Two embodiments are contemplated herein, where in the first embodiment the anterior piece 9 is formed by only one piece, wherein from its part adapted to encircle the ankle and foot it transitions smoothly to a part adapted to encircle the front side of the tibia. This embodiment is shown in Fig. 5. In the second embodiment shown in Fig. 6, the anterior piece 9 is composed of two separate pieces, one adapted to encircle the ankle and foot and the other anterior piece 9 adapted to encircle the front side of the tibia. In these embodiments, the orthosis comprises a second clamping mechanism 9 on the tibial piece 15, through which the upper side of the anterior piece 9 in the first embodiment or the second anterior piece 9 in the second embodiment are connected to the tibial piece 15.

In the alternative embodiments shown in Fig. 5 and Fig. 6, the clamping mechanism 20 is made as a circular cable and a fixing clasp, wherein the cable is fixed to one side of the shell 1 by one side thereof and is adapted to be fixed by the clasp by the other side thereof. The anterior piece 9 at the level of the clamping mechanism 20 includes grooves for fixing the cable, whereby the anterior piece 9 is connected to the fixing mechanism 20 of this embodiment. Different embodiments of the fixing mechanisms can be combined as shown in Fig. 5 and Fig. 6.

The design and production method of the orthosis of the invention will be described in detail herein.

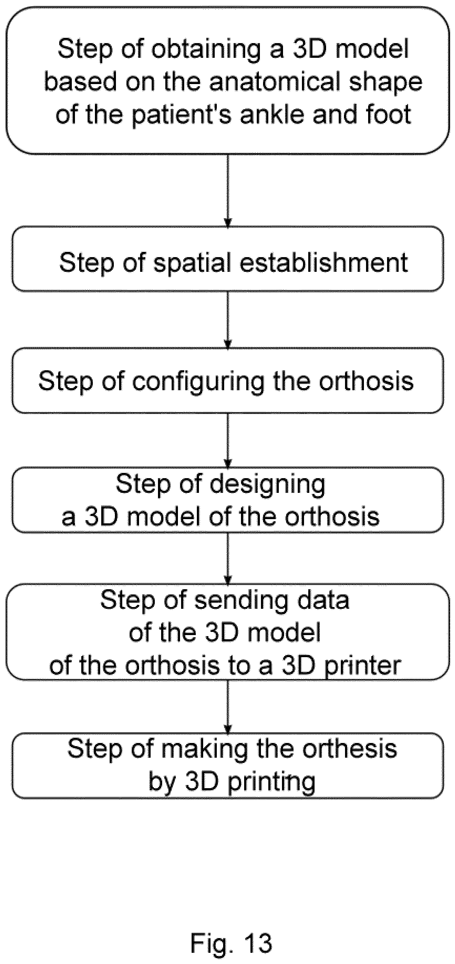

A basic diagram of the design method is shown in Fig. 13. The initial step is the step of obtaining a 3D model based on the anatomical shape of the ankle and foot, wherein this model can be obtained in several ways. The most common way of obtaining the 3D model based on the anatomical shape of the ankle and foot is a direct 3D scan of the patient's foot and ankle. The scan can be performed in unloaded or semi-loaded position of the ankle and foot, depending on the type of pathology or injury of the patient. Preferably, the scan is created in the corrected position of the ankle and foot, when the foot and ankle are oriented to the correct position they should take in the orthosis for the correct treatment of walking pathology or injury. The 3D scan further transforms the data obtained from the 3D scan of the patient's ankle and foot into a polygonal grid of defining points with a density of at least 10 points per cm². Another method is to make a physical model of the ankle and foot, for example from a cast of the patient's ankle and foot, and then 3D scan the created physical model of the ankle and foot. In some cases, it is advisable to adjust the physical model of the ankle and foot before 3D scanning. Other ways of obtaining the 3D model based on the anatomical shape of the ankle and foot are, for example, digitising the inner surface of an existing orthosis or making a physical model of the ankle and foot from the existing orthosis and then digitising the outer surface of the obtained physical model of the ankle and foot. The obtained 3D model based on the anatomical shape of the ankle and foot is then uploaded from the scanning device to a computer device, where it is further processed.

The step of obtaining the 3D model based on the anatomical shape of the ankle and foot may further comprise the step of modifying the 3D model based on the anatomical shape of the ankle and foot at the computer device. The 3D model of the foot and ankle obtained from the scan can be modified and adapted to the patient's needs before further steps, wherein the shape of the orthosis, or the shape of the internal surfaces of the orthosis which are in contact with the patient's foot and ankle, will be directly derived from the resulting modified 3D model based on the anatomical shape of the ankle and foot. For some types of orthoses, a tibia model is also required, wherein the 3D model based on the anatomical shape of the ankle and foot is in these cases a 3D model based on the anatomical shape of the ankle, foot, and tibia.

The next step is the step of spatially establishing the 3D model based on the anatomical shape of the ankle and foot. In this step, the 3D model based on the anatomical shape of the ankle and foot is correctly anatomically oriented in three-dimensional space relative to the reference plane. Another reference element can be used outside the reference plane. The spatial establishment can be done automatically based on a recognition algorithm or manually based on user input. For example, in the case of automatic spatial establishment, specific anatomical regions (e.g., the regions 7, 8 of the medial and lateral ankle, toes, or the region 19 of the heel) may be determined on the 3D model based on the anatomical shape of the ankle and foot, based on which the 3D model is recognised and oriented by the computer. Recognition of the 3D model means the overall recognition of the shapes of the patient's foot and ankle in the 3D model and the correct determination of the anatomical regions on the 3D model. Further, the 3D model based on the anatomical shape of the ankle and foot may be recognised based on the shape and size of the cross-section or circumference of the foot and ankle in different planes on the 3D model. The model recognition can be further achieved by comparing the 3D model with an already established 3D model based on the anatomical shape of the ankle and foot and establishing it based on similarity.

In the case of establishing the 3D model on the basis of the anatomical shape of the ankle and foot based on user input, at least one point from a plurality of points of the 3D model is marked on the computer device via the user input. This reference point may be, for example, the medial ankle, lateral ankle, centre of the heel, the metatarsal head of the big toe or the metatarsal head of the little toe. The computer device then recognises the 3D model based on the position of the reference point and what was marked by the reference point, determines the other anatomical regions of the foot and ankle on the 3D model, and establishes the 3D model relative to the reference plane or other reference element. In addition, an embodiment where the 3D model is manually oriented relative to the reference plane based on user input is also considered, wherein it is also possible to manually mark anatomical regions on the model.

The next step is the step of configuring the orthosis, comprising the design and/or visual embodiment of the orthosis by entering input parameters via the user interface of the computer device. The method of designing and producing the orthosis of the invention further comprises the step of obtaining the input parameters. The step of obtaining the input parameters may also take place during or before the step of spatially establishing or the step of obtaining the 3D model based on the anatomical shape of the tibia and foot, wherein for the proper functioning of the invention, it is irrelevant when the step of obtaining the input parameters is implemented. In the step of configuring the orthosis, the input parameters are then entered via the user interface, or the user interface allows a choice of options, where the input parameter then means the selected option.

The input parameters comprise the dimensions of the patient's foot, where the dimensions of the patient's foot comprise the length of the foot, the distance in the direction of the longitudinal axis of the foot between the outermost point of the heel of the foot and the metatarsal head of the little toe, the distance in the direction of the longitudinal axis of the foot between the outermost point of the heel of the foot and the metatarsal head of the big toe, and the width of the foot at a point between the metatarsal head of the little toe and the metatarsal head of the big toe. Preferably, the foot dimensions are measured directly on the patient's foot and ankle, where it is possible to measure the input parameters at different positions of the foot, e.g., during toe flexion or when loaded, wherein the location of characteristic bones and joints in the foot and ankle can be better recognised, and in some cases it is not necessary to perform an X-ray or ultrasound of the patient's foot and ankle. Such measurements are normally made using a conventional measuring device that comprises a heel support wall and a tread pad on which the units of distance measured from the heel support wall are marked. The patient rests the heel on the heel support wall, wherein the point of contact between the heel support wall and the heel of the patient's foot can be considered the outermost point of the heel of the foot. The width of the foot at the point between the metatarsal head of the little toe and the metatarsal head of the big toe is measured under load. The width of the foot is measured perpendicular to the longitudinal axis of the foot, wherein the foot is flattened during treading or loading and the distance between the metatarsal head of the little toe and the metatarsal head of the big toe is increased. The distance in the direction of the longitudinal axis of the foot between the outermost point of the heel of the foot and the metatarsal head of the little toe is measured based on the flexion of the little toe, and the distance in the direction of the longitudinal axis of the foot between the outermost point of the heel of the foot and the metatarsal head of the big toe is measured based on the flexion of the big toe. During flexion, it is more apparent where the metatarsal heads are located on the patient's foot. When referring to the metatarsal head of the big toe when measuring the dimensions of the foot, it means the intersection of the outer surface of the foot in the region of the metatarsal head of the big toe and the transverse axis of the foot, which essentially passes through the centre of the metatarsal head of the big toe. When referring to the metatarsal head of the little toe when measuring the dimensions of the foot, it means the intersection of the outer surface of the foot in the region of the metatarsal head of the little toe and the transverse axis of the foot, which essentially passes through the centre of the metatarsal head of the little toe. An embodiment is also contemplated where the step of obtaining the 3D model based on the anatomical shape of the ankle and foot comprises obtaining multiple models which represent the foot and ankle of the patient in different positions, for example, during flexion of the big toe, flexion of the little toe, or during treading on the toe, wherein the dimensions of the foot of the patient are measured in the interface of the computer device on these 3D models based on the anatomical shape of the ankle and foot.

The input parameters may also comprise other dimensions of the patient's foot and other parameters used to specify the embodiment of the orthosis. Input parameters may also comprise the type of the orthosis. Preferably, the user interface of the computer device allows selection of the type of the orthosis comprising at least two types of orthoses from the group of an ankle and foot orthosis, an ankle, foot, and back side of the tibia orthosis, and an ankle, foot, and front side of the tibia orthosis. The patient's foot dimensions may further comprise the width of the foot at the heel, the ankle spacing, ankle spacing above the ankles, and ankle height. Furthermore, input parameters may comprise the patient's gender, overall patient height, patient weight, patient age, patient activity level, patient walking pathology, heel height and correction, axial setting of the patient's foot and ankle, type of the 3D model based on the anatomical shape of the ankle and foot (modified, unmodified), specification of the forefoot surface 21, type of the anterior piece 9 (one-piece, two-piece), flexibility of the anterior piece 9, specification of the heel stabiliser, specification of the orthosis insert, type of 3D printing material, location of the flexible regions, rigidity of the flexible regions, embodiment of the clamping mechanism 20, specification of a sensorimotor insert, and specification of marginal curve.

The step of designing a 3D model of the orthosis based on the 3D model based on the anatomical shape of the ankle and foot established in space and the input parameters follows. In this step, the input parameters are processed in the computer device together with the 3D model based on the anatomical shape of the ankle and foot, and then the 3D model of the orthosis is designed. The 3D model of the orthosis is based on the 3D model based on the anatomical shape of the ankle and foot, wherein the shape of the inner walls of the orthosis corresponds to the shape of the 3D model based on the patient's ankle and foot. In terms of size, preferably the inner walls of the orthosis adapted to contact the patient's foot and ankle are not completely identical to the outer walls of the 3D model based on the anatomical shape of the ankle and foot, but the inner walls of the orthosis are slightly oversized. Depending on the input parameters, the dimensions of the inner walls of the orthosis are oversized relative to the 3D model based on the anatomical shape of the ankle and foot by 0.1% to 1%. The specific design and visual embodiment, the external dimensions of the shell 1 and the anterior piece 9, the location of the rigid and flexible regions, the embodiment of the clamping mechanism 20, and other adjustments to the orthosis are further designed based on the input parameters.

Here it will be explained how the various input parameters affect the design of the 3D printed orthosis of the invention. By saying that an orthosis parameter is determined by some input parameter, it is meant that the input parameter is the initial criterion for that orthosis parameter. For example, in the case of foot dimensions, this may mean that a certain orthosis dimension exactly matches a given foot dimension. However, some orthosis parameters may be affected by more than one input parameter. Based on the type of the orthosis, the height of the orthosis is determined, or the level of the patient's lower extremity to which it is encircled by the top part 3 of the shell and the anterior piece 9. For ankle and foot orthoses, the height of the orthosis is generally 1 to 2 cm above the level 25 of the ankles, and for higher orthoses that also encircle the tibia, specifically ankle, foot, and back side of the tibia orthoses and ankle, foot, and front side of the tibia orthoses, the height may vary depending on the specific requirements for the patient. Based on the width of the foot at the point between the metatarsal head of the little toe and the metatarsal head of the big toe, the distance between medial part 4 and lateral part 5 is determined at the level 23 of the metatarsal heads. Based on the width of the foot at the point between the metatarsal head of the little toe and the metatarsal head of the big toe, the width of the tread surface of the bottom part 2 of the shell at the level 23 of the metatarsal heads is also determined.

Based on the distance in the direction of the longitudinal axis of the foot between the outermost point of the heel of the foot and the metatarsal head of the little toe, the length of the lateral part 5 is determined, and based on the distance in the direction of the longitudinal axis of the foot between the outermost point of the heel of the foot and the metatarsal head of the big toe, the length of medial part 4 is determined. By the length of medial part 4 and lateral part 5 is meant their dimension in the direction of the longitudinal axis of the foot. The length of the bottom part 2 of the shell is determined based on the length of the patient's foot when the bottom part 2 of the shell comprises the forefoot surface 21. In an embodiment where the bottom part 2 of the shell does not include the forefoot surface 21, the length of the bottom part 2 of the shell is determined based on the distance in the direction of the longitudinal axis of the foot between the outermost point of the heel of the foot and the metatarsal head of the little toe and the distance in the direction of the longitudinal axis of the foot between the outermost point of the heel of the foot and the metatarsal head of the big toe.

The location of the front edge of the anterior piece is determined by the level 23 of the metatarsal heads. The width of the front edge of the anterior piece 9 is determined based on the circumference of the foot at the level 23 of the metatarsal heads and based on the gap between the medial and lateral parts 4, 5 at the level 23 of the metatarsal heads. Preferably, the lateral part 5 overlaps approximately 1 to 2 cm of the lateral side 13 of the anterior piece over the entire length of the anterior piece 9 and the medial part 4 overlaps approximately 1 to 2 cm of the medial side 12 of the anterior piece over the entire length of the anterior piece 9. The length of the anterior piece 9 is determined based on the height of the orthosis, wherein the height of the top part 3 of the shell or tibial piece 15 corresponds to the upper edge of the anterior piece 9. The anterior piece 9 follows the shape of the foot, ankle, and tibia, which have a shape resembling the letter "L", and the marginal edges of the anterior piece 9 are thus called the upper edge of the anterior piece 9 and the front edge of the anterior piece 9.

The width of the upper edge of the anterior piece 9 is determined based on the circumference of the patient's lower extremity at the level of the upper edge of the anterior piece 9 and based on the width of the gap between the medial and lateral parts 4, 5 at the level of the upper edge of the anterior piece 9. The shape of the upper edge of the anterior piece 9 and the front edge of the anterior piece 9 corresponds to the shape of the foot, ankle, or tibia at the point of the given edge. Wherein the shape of the anterior piece 9 corresponds to the shape of the anterior region 10 of the foot, ankle, or tibia between the upper edge and the front edge of the anterior piece 9. The anterior piece 9 encircles 20% to 60% of the length of the circumference of the foot, ankle, or tibia, depending on the distance between the medial part 4 and the lateral part 5 in the anterior region 10.

The rigidity and location of the rigid regions is determined based on the patient's weight, activity level, walking pathology, patient height, and axial setting of the foot and ankle, where, for example, the size and rigidity of the rigid regions increases with increased patient weight, height, or activity level, and based on the type of walking pathology, the rigidity and location of the rigid region on the bottom part 2 of the shell are determined. The rigidity and location of the flexible regions of the first type is determined based on the location and nature of critical regions of the ankle and foot, such as the regions 7, 8 of the ankles or other points where the bones project to a greater extent, or sensitive regions of the ankle and foot of the wearer of the orthosis, wherein these regions may be marked based on user input or recognised automatically by the computer device from the 3D model based on the anatomical shape of the ankle and foot. The rigidity and location of the flexible regions of the second type are determined based on the location and size of the instep 16 region, the type of the anterior piece 9, patient height, patient weight, and patient activity level. The rigidity of the forefoot surface 21 is determined based on patient height, patient weight, patient activity level, and walking pathology. The rigidity and location of the flexible and rigid regions can also be directly specified or modified based on user input.

The specification of the orthosis model is further sent to the 3D printer for production in the step of sending the data of the 3D model of the orthosis to the 3D printer. In this step, the model of the orthosis together with the instructions for its printing can be processed within the computer device into a specific form of 3D orthosis model data, which comprise a specific set of instructions for the 3D printer, resulting in the designed orthosis. One possible format for these instructions is the so-called G code or similar formats. In an alternative embodiment, the 3D orthosis model data may directly comprise a 3D orthosis model, wherein the 3D comprises the 3D orthosis model processing module in which the orthosis model is analysed and subsequently transformed into a set of instructions for the 3D printer. In the final step of making the orthosis by 3D printing, the 3D printer uses 3D printing technology to produce the orthosis based on the data of the 3D orthosis model.

The computer device includes in its memory a software application that comprises an interactive configurator and a data export module. The interactive configurator comprises a module for collecting anatomical and/or clinical data, a module for selecting the corresponding type of orthosis, a module for design modifications through which input parameters are entered via the user input of the computer device. In another embodiment, the interactive configurator further comprises a system for modifying an input model of the ankle and foot of the patient. The software application may further comprise a module for inputting the measured data within the model for collecting anatomical and/or clinical data, module for visually configuring the orthosis, module for manually spatially establishing the orthosis comprising a module for marking reference points, and a module for displaying and adjusting the marginal curve. The scanner is communicatively connected to the computer device via a cable or wireless connection. The computer device further comprises user input in the form of, for example, a touch screen, touchpad, mouse, and/or keyboard. The computer device is further communicatively connected to the 3D printer by a cable or wireless connection.

### List of Reference Signs

- 1: - shell
- 2: - bottom part of the shell
- 3: - top part of the shell
- 4: - medial part
- 5: - lateral part
- 6: - plantar region of the foot
- 7: - the medial ankle region
- 8: - the lateral ankle region
- 9: - anterior piece
- 10: - anterior region
- 11: - central part of the anterior piece
- 12: - medial side of the anterior piece
- 13: - lateral side of the anterior piece
- 14: - cutout
- 15: - tibial piece
- 16: - the instep region
- 17: - posterior part
- 18: - posterior region
- 19: - region of the heel
- 20: - clamping mechanism
- 21: - forefoot surface
- 22: - forefoot of the foot
- 23: - level of the metatarsal heads
- 24: - integrated grip
- 25: - level of the ankles
- 26: - midfoot of the foot
- 27: - hindfoot of the foot

## Claims

1. A 3D printed ankle and foot orthosis comprising a 3D printed shell (1), wherein the shell (1) comprises a bottom part (2) and a top part (3), where the top part (3) forms with the bottom part (2) a single piece and the top part (3) comprises a medial part (4) and a lateral part (5), the bottom part (2) is adapted to support a plantar region (6) of the foot, wherein the medial part (4) is shaped to contact the medial side of the ankle and foot and extends over the medial ankle region (7) and the lateral part (5) is shaped to contact the lateral side of the ankle and foot and extends over the lateral ankle region (8), wherein the bottom part (2) comprises a rigid region and at least one of the medial part (4) and the lateral part (5) comprises a flexible region, **characterised in that** it further comprises an anterior piece (9) located between the medial part (4) and the lateral part (5), wherein the anterior piece (9) is adapted to encircle an anterior region (10) of the foot and ankle between the medial part (4) and the lateral part (5), and that the anterior piece is formed of a single material, wherein the medial side (12) of the anterior piece and the lateral side (13) of the anterior piece each comprise at least one transverse cutout (14) from the margin of the medial side (12) of the anterior piece and the lateral side (13) of the anterior piece towards the central part (11) of the anterior piece, wherein the transverse cutouts (14) are adapted to increase the flexibility of the central part (11) of the anterior piece at a point between the transverse cutouts (14).

2. The 3D printed ankle and foot orthosis of claim 1, **characterised in that** the anterior piece (9) comprises the longitudinal central part (11), the medial side (12) of the anterior piece, and the lateral side (13) of the anterior piece, wherein the central part (11) comprises a rigid region and the lateral side (13) of the anterior piece and the medial side (12) of the anterior piece each comprise a flexible region.

3. The 3D printed ankle and foot orthosis of claims 1 or 2, **characterised in that** the difference in rigidity between the flexible region and the rigid region is provided by at least one of the following options:
- The flexible region comprises a plurality of openings, wherein the flexible region comprises a greater proportion of the volume representation of the openings relative to the volume representation of the 3D printed structure filling the space between the shaped openings than the rigid region,
- the wall thickness of the flexible region at its thinnest point is less than the wall thickness of the rigid region at its thinnest point,
- the flexible region is formed by one wall while the rigid region comprises at least two interconnected walls, or
- the rigid region comprises reinforcing projections.

4. The 3D printed ankle and foot orthosis of any one of the preceding claims, **characterised in that** the medial part (4) of the top part (3) of the shell and the medial side (12) of the anterior piece partially overlap and the lateral part (5) of the top part (3) of the shell and the lateral side (13) of the anterior piece partially overlap.

5. The 3D printed ankle and foot orthosis of any one of the preceding claims, **characterised in that** the medial side (12) of the anterior piece and the lateral side (13) of the anterior piece each comprise at least two transverse cutouts (14) from the margins of the medial side (12) and the lateral side (13) of the anterior piece towards the central part (11) of the anterior piece.

6. The 3D printed ankle and foot orthosis of any one of the preceding claims, **characterised in that** the wall thickness of the anterior piece (9) is greater in the central part (11) of the anterior piece than in the medial side (12) and lateral side (13) of the anterior piece.

7. The 3D printed ankle and foot orthosis of any one of the preceding claims, **characterised in that** the wall thickness of the anterior piece (9) in the central part (11) of the anterior piece varies in the longitudinal direction.

8. The 3D printed ankle and foot orthosis of any one of the preceding claims, **characterised in that** it comprises a tibial piece (15) at least partially encircling the tibia, wherein the anterior piece (9) at least partially encircles the tibia.

9. The 3D printed ankle and foot orthosis of any one of the preceding claims, **characterised in that** the top part (3) of the shell comprises a flexible region in at least one of the regions: the lateral ankle region (8), the medial ankle region (7), and the instep (16) region.

10. The 3D printed ankle and foot orthosis of any one of the preceding claims, **characterised in that** the top part (3) of the shell in the posterior region (18) over the region (19) of the heel comprises a gap separating the medial part (4) and the lateral part (5).

11. The 3D printed ankle and foot orthosis of any one of the preceding claims, **characterised in that** it includes a clamping mechanism (20) for tightening the anterior piece (9) over the anterior region (10) of the foot and ankle by means of the medial part (4) and/or lateral part (5), wherein the shell (1) is connected to the anterior piece (9) via the clamping mechanism (20).

12. The 3D printed ankle and foot orthosis of any one of the preceding claims, **characterised in that** the bottom part (2) of the shell includes a forefoot surface (21) adapted to support the forefoot (22) of the foot from the level (23) of the metatarsal heads towards the toe of the foot, wherein the forefoot surface (21) has a variable thickness decreasing towards the toe of the foot.

13. A method of production of the 3D printed ankle and foot orthosis of any one of the preceding claims comprising the following steps:
1) a step of obtaining a 3D model based on the anatomical shape of the patient's ankle and foot,
2) a step of spatially establishing the 3D model based on the anatomical shape of the patient's ankle and foot, either automatically or based on user input by marking at least one point from a plurality of points of the 3D model based on the anatomical shape of the patient's ankle and foot,
3) a step of configuring the orthosis, comprising specification of the design embodiment of the orthosis by entering input parameters via a user interface,
4) a step of designing a 3D model of the orthosis based on the 3D model based on the anatomical shape of the ankle and foot established in space and the input parameters,
5) a step of sending the data of the 3D model of the orthosis to a 3D printer,
6) a step of making the orthosis by 3D printing,
**characterised in that**
the input parameters comprise dimensions of the patient's foot comprising the distance in the direction of the longitudinal axis of the foot between the outermost point of the heel of the foot and the metatarsal head of the little toe, the distance in the direction of the longitudinal axis of the foot between the outermost point of the heel of the foot and the metatarsal head of the big toe, and the width of the foot at a point between the metatarsal head of the little toe and the metatarsal head of the big toe, wherein in the step of designing the 3D model of the orthosis, the dimensions of the shell (1) and the anterior piece (9) are determined based on the length of the foot, the distance in the direction of the longitudinal axis of the foot between the outermost point of the heel of the foot and the metatarsal head of the little toe, the distance in the direction of the longitudinal axis of the foot between the outermost point of the heel of the foot and the metatarsal head of the big toe, and the width of the foot at the point between the metatarsal head of the little toe and the metatarsal head of the big toe, and the 3D model based on the anatomical shape of the patient's ankle and foot.

14. The method of production of the 3D printed ankle and foot orthosis of claim 13, **characterised in that** the width of the foot at the point between the metatarsal head of the little toe and the metatarsal head of the big toe is measured with the foot loaded, the distance in the direction of the longitudinal axis of the foot between the outermost point of the heel of the foot and the metatarsal head of the little toe is measured based on the flexion of the little toe, and the distance in the direction of the longitudinal axis of the foot between the outermost point of the heel of the foot and the metatarsal head of the big toe is measured based on the flexion of the big toe.

15. The method of production of the 3D printed ankle and foot orthosis of claims 13 or 14, **characterised in that** in the step of designing the 3D model of the orthosis, the distance between the medial part (4) and the lateral part (5) at the level (23) of the metatarsal heads is determined based on the width of the foot at the point between the metatarsal head of the little toe and the metatarsal head of the big toe, the length of the lateral part (5) is determined based on the distance in the direction of the longitudinal axis of the foot between the outermost point of the heel of the foot and the metatarsal head of the little toe, and the length of the medial part (4) is determined based on the distance in the direction of the longitudinal axis of the foot between the outermost point of the heel of the foot and the metatarsal head of the big toe.

16. The method of production of the 3D printed ankle and foot orthosis of claims 13 to 15, **characterised in that** in the step of designing the 3D model of the orthosis, the length of the bottom part (2) of the shell is determined based on the length of the patient's foot or based on the distance in the direction of the longitudinal axis of the foot between the outermost point of the heel of the foot and the metatarsal head of the little toe and the distance in the direction of the longitudinal axis of the foot between the outermost point of the heel of the foot and the metatarsal head of the big toe.

17. The method of production of the 3D printed ankle and foot orthosis of claims 13 to 16, **characterised in that** the step of configuring the orthosis comprises selecting the type of the orthosis comprising at least two types of orthoses from the group of: an ankle and foot orthosis, an ankle, foot, and the back side of the tibia orthosis, and an ankle, foot, and the front side of the tibia orthosis, wherein based on the selected type of the orthosis, the height of the orthosis is determined.

18. The method of production of the 3D printed ankle and foot orthosis of claims 13 to 17, **characterised in that** the location of the front edge of the anterior piece (9) is determined based on the level (23) of the metatarsal heads, the width of the front edge of the anterior piece (9) is based on the circumference of the foot at the level (23) of the metatarsal heads, the length of the anterior piece (9) is determined based on the type of the orthosis, and the width of the upper edge of the anterior piece (9) is based on the circumference of the patient's lower extremity at the level of the upper edge of the anterior piece (9), wherein the anterior piece (9) encircles 20% to 60% of the length of circumference of the foot, ankle, or tibia.
